# EUROPÄISCHE PATENTANMELDUNG

(11) **EP 1 621 201 A1**
(43) Veröffentlichungstag der Anmeldung: **01.02.2006**
(21) Anmeldenummer: 04017845.1
(22) Anmeldetag: 28.07.2004
(51) Int. Cl.: A61K 31/7016, A61P 25/16, A61P 25/28, A61P 9/10

(54) **Hesperidin zur Prophylaxe und Behandlung von Erkrankungen des zentralen Nervensystems, insbesondere Morbus Alzheimer, Morbus Parkinson, zur Therapie nach Schlaganfall sowie von chronischen Schmerzzuständen**

(71) Anmelder: NeuroCode AG, 35440 Linden (DE)
(72) Erfinder: Dimpfel, Wilfried, Prof. Dr., 35415 Pohlheim (DE)

(57) **Zusammenfassung**

Die vorliegende Erfindung betrifft die Verwendung von Hesperidin (bzw. als Aglykon Hesperetin) oder seinen pharmazeutisch annehmbaren Salzen oder Komplexen (Chalkonen) zur Prophylaxe und Behandlung von Erkrankungen des zentralen Nervensystems, inbesondere von neurodegenerativen Erkrankungen wie beispielsweise Morbus Alzheimer und Morbus Parkinson, sowie zur Therapie der Folgen nach Schlaganfall. Darüberhinaus besitzt Hesperidin zentralnervös bedingte analgetische Eigenschaften.

## Beschreibung

### Technisches Gebiet

Die vorliegende Erfindung betrifft die Verwendung von Hesperidin (bzw. als Aglykon Hesperetin) oder seinen pharmazeutisch annehmbaren Salzen oder Komplexen (Chalkonen) zur Prophylaxe und Behandlung Erkrankungen des zentralen Nervensystems, insbesondere von neurodegenerativen Erkrankungen wie beispielsweise Morbus Alzheimer und Morbus Parkinson, sowie zur Therapie der Folgen nach Schlaganfall. Darüberhinaus besitzt Hesperidin zentralnervös bedingte analgetische Eigenschaften.

### Hintergrund der Erfindung

Hesperidin, auch als Hesperetin 7-rhamnoglucosid, bekannt, hat folgende Summenformel: C28H34O15 und die allgemeine Formel:

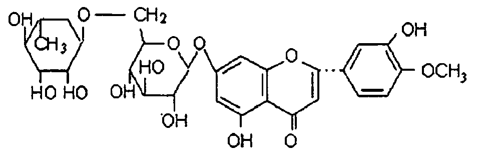

Hesperidin (Aglykon Hesperetin) ist ein pflanzlicher Inhaltsstoff, der vorwiegend in Zitrusfrüchten (Orangen), in geringen Mengen auch z.B. in Valeriana officinalis vorkommt. Hesperidin wird auch als Bestandteil von Kombinationspräparaten (Cyclo 3 fort, Daflon) zur Behandlung venöser Gefäßerkrankungen verwendet. Auf der Basis der bisher bekannt gewordenen Untersuchungen war eine Wirkung von Hesperidin bei Erkrankungen des zentralen Nervensystems, insbesondere von neurodegenerativen Erkrankungen wie z.B. auf das Krankheitsbild der Alzheimerschen Erkrankung bzw. Morbus Parkinson, sowie im Rahmen der Rekonvaleszens nach Schlaganfall nicht zu erwarten gewesen. Ebenso gab es keine Hinweise auf eine zentrale analgetische Wirkung. Erst die Anwendung der völlig "bias"-freien Untersuchung im Tele-Stereo-EEG der Ratte erbrachte den Beweis des Vorhandenseins einer derartigen zentralnervösen Wirkung.

Die derzeit üblichen Arzneimittel zur Behandlung von Erkrankungen des zentralen Nervensystems, insbesondere von neurodegenerativen Erkrankungen (Morbus Alzheimer, Morbus Parkinson) sowie der Folgen eines Schlaganfalls sind unzufriedenstellend. Zudem ist eine Beeinflussung des cholinergen Transmittersystems, das die Basis der meisten bisherigen therapeutischen Ansätze z.B. beim Morbus Alzheimer darstellt, mit erheblichen Nebenwirkungen sowohl im Bereich des Herz-Kreislaufsystems wie auch des zentralen Nervensystems verbunden (Kopfschmerzen, Schwindel usw.). Es besteht daher ein großer Bedarf nach einer Prophylaxe bzw. einem verbesserten Arzneimittel mit einer guten therapeutischen Wirksamkeit bei möglichst geringer Nebenwirkungsrate. Insbesondere gilt dies für die Prophylaxe, z.B. über die Verabreichung von Nahrungsergänzungsmitteln, wo bislang keine wissenschaftlich abgesicherte Anwendung in Bereich dieser Erkrankungen gefunden wurde (McDaniel MA, Maier SF und Einstein GO "Brain-specific" nutrients: a memory cure? Nutrition (2003) 19: 957-75).

### Zusammenfassung der Erfindung

Diese Aufgabe wurde überraschenderweise durch die Verwendung von Hesperidin (Aglykon Hesperetin) bzw. deren pharmazeutisch annehmbaren Salzen als Wirkstoff für die Herstellung eines Arzneimittels zur Behandlung von Erkrankungen des zentralen Nervensystems, insbesondere von neurodegererativen Erkrankungen wie z.B Morbus Alzheimer und Morbus Parkinson, sowie zur Behandlung der durch einen Schlaganfall ausgelösten zentralnervösen Ausfallserscheinungen, gelöst. Hierbei ist die zentralnervös bedingte analgetische Wirkung von Hesperidin von unterstützender Wirkung, kann aber auch allgemein zur Behandlung von Schmerzzuständen separat eingesetzt werden.

### Kurze Beschreibung der Abbildungen

Fig. 1 zeigt die Veränderungen der EEG-Frequenzen bei der Ratte nach Gabe klassischer Arzneimittel zur Behandlung von Erkrankungen des zentralen Nervensystems, insbesondere von neurodegenerativen Erkrankungen (Memantine, Tacrin, Piracetam), Schmerzen (Metamizol) im Vergleich zu Hesperidin.

Fig. 2 zeigt eine Diskriminanzanalyse der EEG-Frequenzänderungen nach Gabe von Medikamenten für unterschiedliche Indikationen im Vergleich zu solchen, wie sie zur Behandlung von neurodegenerativen Erkrankungen oder zur Behandlung der Folgen eines Schlaganfalls (z.B. Amphetamin) verwendet werden (s. zentraler Kreis).

Fig. 3a und 3b zeigen die zeitabhängigen Veränderungen der EEG-Frequenzen bei der Ratte nach Gabe von 80 bzw. 160 mg/kg Hesperidin i.p.. Insbesondere die delta, theta und alpha2 Frequenzen nehmen statistisch signifikant und dauerhaft ab.

### Detaillierte Beschreibung der Erfindung

Bei Untersuchungen der Veränderungen der EEG-Frequenzen der Ratte nach Gabe von Hesperidin wurde überraschenderweise gefunden, dass das Präparat eine Wirkung im Gehirn besitzt, die bei der Behandlung von Erkrankungen des Gehirns, insbesondere von neurodegenerativen Erkrankungen, Schmerz sowie in der Rekonvaleszenz nach Schlaganfall nützlich ist. Ebenso zeigte sich, daß Hesperidin über eine zentralnervös bedingte analgetische Wirkkomponente verfügt.

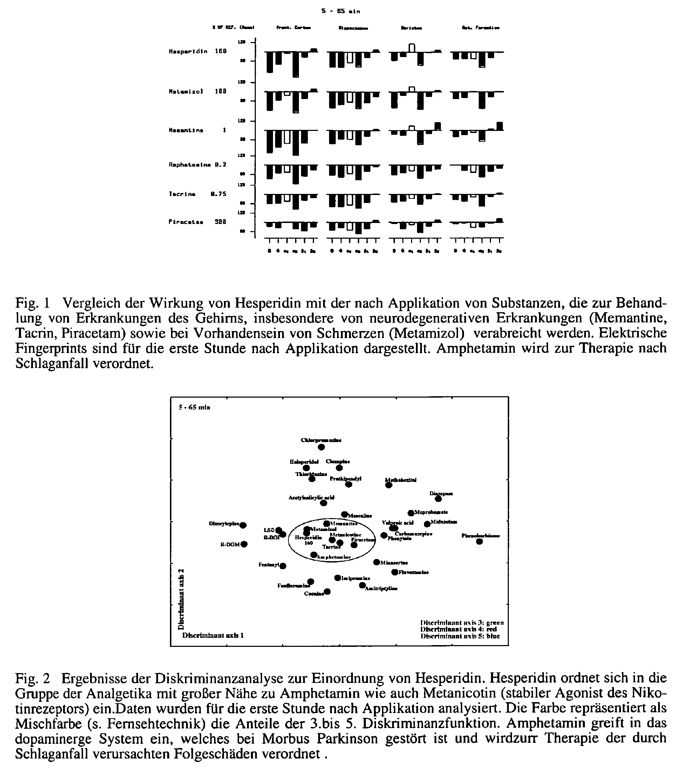

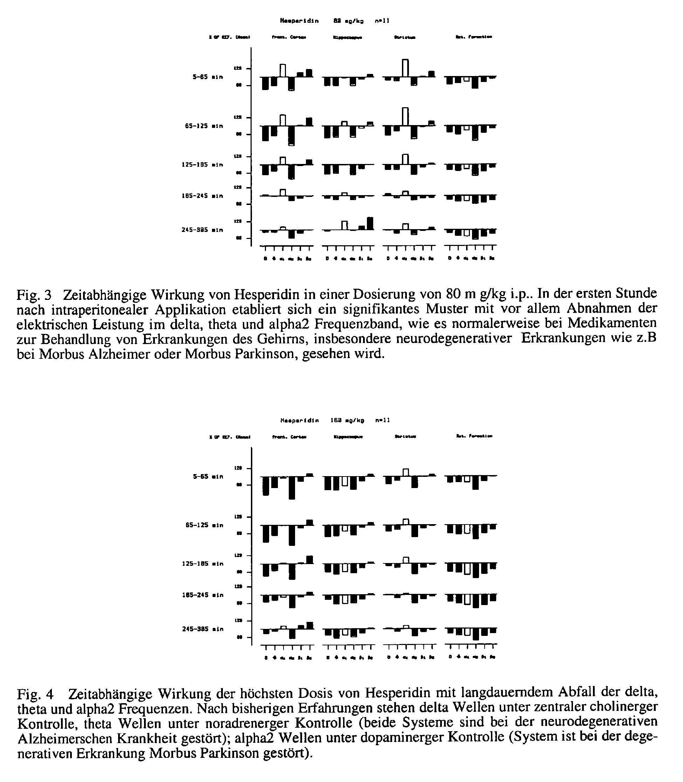

Hesperidin zeigt überraschenderweise im Modell Tele-Stereo-EEG bei Ratten Veränderungen der EEG-Frequenzen, wie sie nach Gabe klassischer Medikamente zur Behandlung neurodegenerativer Erkrankungen wie Morbus Alzheimer und Morbus Parkinson, sowie zur Behandlung von Schlaganfallpatienten wie auch im Rahmen einer Schmerzbehandlung in diesem Modell gemessen werden. Die Veränderungen, wie sie nach Gabe klassischer Medikamente sowie nach Gabe von Hesperidin auftraten, sind in Fig. 1 gezeigt. Auf Grund inzwischen bekannt gewordener Korrelationen zwischen EEG Frequenzen und zentralen Neurotransmitteraktivitäten kann auf eine Änderung der cholinergen (delta Wellen), noradrenergen (theta Wellen) und dopaminergen (alpha2 Wellen) Aktivitätsänderung geschlossen werden. Diese Systeme sind bei den genannten Erkrankungen in erster Linie in ihrer Funktion gestört. Aus der in Fig. 2 gezeigten Diskriminanzanalyse ist offensichtlich, dass sich der charakteristische elektrische Fingerprint, der für klassische Medikamente zur Behandlung neurodegenerativer Erkrankungen, Schmerz sowie nach Schlaganfall typisch ist, auch im Muster von Hesperidin in signifikanter Weise wiederfindet. Im Vergleich dazu zeigen Fig. 3a und 3b die zeitabhängigen Veränderungen der EEG-Frequenzen nach Gabe von Hesperidin wie sie gemäß Beispiel 1 gemessen wurden. Aus der Erkenntnis, dass Hesperidin die gleichen charakteristischen Veränderungen der EEG-Frequenzen hervorruft wie üblicherweise Arzeimittel, die zur Behandlung neurodegenerativer Erkrankungen wie Morbus Alzheimer oder Morbus Parkinson, Schmerz sowie nach Schlaganfall und im Bereich Schmerztherapie eingesetzt werden, kann gefolgert werden, dass Hesperidin bei der Behandlung in denselben Indikationen wirksam ist. Daß Medikamente, die in der gleichen Indikation eingesetzt werden, auch gleichartige EEG Veränderungen hervorrufen, konnte von Dimpfel anhand von mehr als 40 Referenzsubstanzen für 8 verschiedene Indikationen gezeigt werden (Dimpfel W Preclinical data base of pharmaco-specific rat EEG fingerprints (Tele-Stereo-EEG). Eur J Med Res (2003) 8: 199-207).

Erfindungsgemäß wird Hesperidin (bzw. sein Aglykon Hesperetin) eingesetzt. Diese Substanz, auch als Hesperetin 7-rhamnoglucosid, bekannt, hat folgende Summenformel: C28H34O15 bzw. allgemeine Formel:

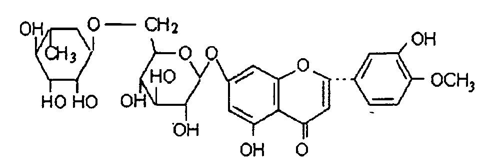

Hesperidin kann erfindungsgemäß auch in Form seiner pharmazeutisch annehmbaren Salze oder Komplexe eingesetzt werden. Die Herstellung solcher Salze erfolgt in an sich bekannter Weise. Als Salzbildner kommen alle üblichen pharmazeutisch annehmbaren Säuren bzw. Anionen in Frage. Insbesondere können auch Chalkone verwendet werden. Auch eine Kopplung an Glucuronsäure kann verwendet werden. Insbesondere kann auch Hesperidin (Glycosid) ohne seine Zuckerkomponente als Hesperetin (Aglycon) verwendet werden.

Die erfindungsgemäßen Hesperidin oder Hesperetin als Wirkstoff enthaltenden Nahrungsergänzungsmittel bzw. Arzneimittel werden bevorzugt oral verabreicht. Es ist jedoch auch eine Verabreichung auf anderem Wege, z.B. peroral, topisch, parenteral, intravenös, intramuskulär, subkutan, nasal, inhalativ, rektal oder transdermal möglich.

Zur Verabreichung kann das erfindungsgemäße Arzeimittel, das als Wirkstoff Hesperidin oder Hesperetin enthält, z.B. in Form von Tabletten, Kapseln, Pillen, Dragees, Granulaten, Suppositorien, Pellets, Lösungen, Dispersionen formuliert werden, wobei der Wirkstoff optional mit pharmazeutisch annehmbaren Hilfs- und Trägerstoffen kombiniert werden kann.

Liegt das erfindungsgemäße Arzeimittel in Form einer Lösung vor, so enthält diese bevorzugt 0.5 bis 20 Gew.-%, besonders bevorzugt 1 bis 10 Gew.-% des Wirkstoffs.

Die erfindungsgemäßen Arzeimittel, die Hesperidin oder Hesperetin als Wirkstoff enthalten, werden normalerweise gemäss herkömmlichen Methoden hergestellt und in einer pharmazeutisch geeigneten Form verabreicht.

Zum Beispiel können die festen oralen Formen zusammen mit dem Wirkstoff Streckstoffe, z.B. Lactose, Dextrose, Saccharose, Cellulose, Maisstärke oder Kartoffelstärke; Gleitmittel, z.B. Silikat, Talk, Stearinsäure, Magnesium- oder Calciumstearat und/oder Polyethylenglykole; Bindemittel, z.B. Stärken, Gummi arabicum, Gelatine, Methylcellulose, Carboxymethylcellulose oder Polyvinylpyrrolidon: Aufschussmittel, z.B. Stärke, Alginsäure, Alginate oder Natriumstärkeglykolate, aufschäumende Mischungen; Farbstoffe; Süßungsmittel; Benetzungsmittel, wie Lecithin, Polysorbate, Laurylsulfate: um im allgemeinen nicht-toxische und pharmakologisch inaktive Substanzen, die in pharmazeutischen Formulierungen verwendet werden, enthalten.

Die pharmazeutischen Zubereitungen können in bekannter Weise hergestellt werden, z.B. mittels Mischen, Granulieren, Tablettieren, Zuckerbeschichtungs- oder Überzugsbeschichtungsverfahren.

Die flüssigen Dispersionen zur oralen Verabreichung können z.B. Sirupe, Emulsionen und Suspensionen sein.

Der Sirup kann als Träger z.B. Saccharose oder Saccharose mit Glycerin und/oder Mannitol und/oder Sorbitol enthalten.

Die Suspensionen und die Emulsionen können als Träger z.B. ein natürliches Harz, Agar, Natriumalginat, Pectin, Methylcellulose, Carboxymethylcellulose oder Polyvinylalkohol enthalten.

Die Suspensionen oder Lösungen für intramuskuläre Injektionen können zusammen mit dem Wirkstoff einen pharmazeutisch annehmbaren Träger, z.B. steriles Wasser, Olivenöl, Ehyloleat, Glykole, z.B. Propylenglykol, und, falls gewünscht, eine geeignete Menge an Lidocain-Hydrochlorid enthalten.

Die Lösungen zur intravenösen Injektion oder Infusion können als Träger z.B. steriles Wasser enthalten oder sie können bevorzugt in Form von sterilen, wässrigen, isotonsichen Salzlösungen vorliegen.

Die Suppositorien können zusammen mit dem Wirkstoff einen pharmazeutisch annehmbaren Träger, z.B. Kakaobutter, Polyethylenglykol, einen Polyoxyethylensorbitol-Fettsäureester oder Lecithin enthalten.

Zusammensetzungen für die topische Applikation, z.B. Cremes, Lotionen oder Pasten, können durch Mischen des Wirkstoffs mit einem herkömmlichen ölhaltigen oder emulgierenden Träger hergestellt werden.

Die Dosierungseinheit des Arzeimittels kann beispielsweise enthalten:
bei peroralen Arzneiformen:
bevorzugt 800 bis 2000 mg, besonders bevorzugt 900 bis 1200 mg, Wirkstoff pro Tagesdosis.
   Die Tagesdosis kann beispielsweise in 1 bis 3 Einzeldosen, vorzugsweise in einer Einzeldosis, täglich verabreicht werden.
bei parenteralen Arzeiformen (zum Beispiel intravenös, subkutan, intramuskulär):
bevorzugt 200 bis 600 mg, besonders bevorzugt 300 mg, Wirkstoff pro Tagesdosis.
   Die Tagesdosis kann beispielsweise in 1 bis 3 Einzeldosen, vorzugsweise in einer Einzeldosis, täglich verabreicht werden.
bei Arzneiformen zur rektalen Applikation:
bevorzugt 400 bis 800 mg, besonders bevorzugt 600 mg, Wirkstoff pro Tagesdosis.
   Die Tagesdosis kann beispielsweise in 1 bis 3 Einzeldosen, vorzugsweise in einer Einzeldosis, täglich verabreicht werden.
bei Arzneiformen zur Applikation auf die Haut und Schleimhäute (z.B.
   Lösungen, Lotionen, Emulsionen, Salben usw.):
bevorzugt 400 bis 800 mg Wirkstoff, besonders bevorzugt 600 mg, Wirkstoff pro Einzeldosis.
   Die Tagesdosis kann beispielsweise in 1 bis 6 Einzeldosen, vorzugsweise in 1 -3 Einzeldosen, täglich verabreicht werden.
als Nahrungsergänzungsmittel:
bevorzugt 200 bis 600 mg Wirkstoff, besonders bevorzugt 400 mg als Zugabe zu Getränken.

Bei Verwendung eines pharmazeutisch annehmbaren Salzes muss die verwendete Menge in dem Fachmann bekannter Weise entsprechend angepaßt werden.

### Testverfahren (Tele-Stereo-EEG)

Die Veränderungen der EEG-Frequenzen nach intraperitonealer Gabe von Salzlösung (Kontrolle) bzw. einer Dosis von Hesperidin (80 und 160 mg/kg Körpergewicht) wurden bestimmt.

Die Untersuchungen wurden analog der durch W. Dimpfel beschriebenen Methode (Dimpfel W Preclinical data base of pharmaco-specific rat EEG fingerprints (Tele-Stereo-EEG). Eur J Med Res (2003) 8: 199-207) folgendermaßen durchgeführt:

Zwölf männlichen erwachsenen Fischer-344 Ratten (Tag-Nacht konvertiert) wurden im Alter von 6 Monaten 4 bipolar konzentrische Elektroden zusammen mit einem Mikrostecker auf einer gemeinsamen Basisplatte implantiert. Der Stecker diente der Aufnahme eines 4-Kanal-Senders zur telemetrischen Übertragung der aus frontalem Kortex, Hippokampus, Striatum und Formation Reticularis abgeleiteten Feldpotentiale. Die Signale wurden auf einem Computer System (Software "EEG-Analyse", Betriebssystem OS Science, Laborrechner "LabTeam" der Firma MediSyst, Linden, DE) in Echtzeit einer Fast-Fourier-Transformation unterworfen und die Leistungsdichtespektren jeweils über 15 bis 60 Minuten gemittelt. Die Unterteilung der Spektren in 6 verschiedene Frequenzbereiche erlaubte die Erfassung pharmako-spezifischer Veränderungen in Bezug auf die jeweils vor Applikation gemessenen Vorwerte innerhalb dieser Frequenzbänder.

Zum Applikationsprotokoll der Substanzen: die Substanzen wurden i.p. 45 Minuten nach Beginn der Messungen (Vorwert) injiziert. Fünf Minuten später wurden die Messungen wieder gestartet, mindestens über die nächsten 5 Stunden kontinuierlich analysiert und in 60-minütigen Perioden zusammengefaßt. Die Testsubstanz wurde in einer Dosierung von 80 und 160 mg/kg appliziert. Die experimentelle Serie wurde mit der Injektion von Salzlösung (Kontrolle), die zu keiner auffälligen Änderung führte, begonnen.

Der statistische Vergleich der Versuche zu den Ergebnissen, die nach Gabe von Salzlösung gemessen wurde, erfolgte mit Hilfe einer multivariaten Analyse nach Ahrens und Läuter (siehe H. Ahrens, J. Läuter "Mehrdimensionale Varianzanalyse" (1974), Akademie Verlag, Berlin) auf der Basis der Veränderungen innerhalb der einzelnen Frequenzbänder in allen Hirnregionen als Variablen.

Die Verabreichung von Salzlösung führte kaum zu Veränderungen der elektrischen Aktivität (µV²/ Ω) im Vergleich zu den Vorphasenwerten.

Die Verabreichung von Hesperidin führte dosis-abhängig zu deutlichen Veränderungen der Leistungsdichte in den einzelen Hirnregionen. Nach Gabe von 80 und 160 mg/kg entwickelte sich ein stabiles, statistisch signifikantes Muster, welches über längere Zeit zu beobachten war. Bereits in der ersten Stunde nach Verabreichung von 160 mg/kg Hesperidin wurde ein Abfall in den delta, theta und alpha2 Frequenzen beobachtet. Dieses Muster wurde bei dieser Dosis über die gesamte Versuchszeit von 5 Stunden beobachtet. Die Veränderungen waren statistisch mindestens auf dem P < 5%-Level signifikant.

Die i.p. Verabreichung von Hesperidin als Einzeldosis führt zu Veränderungen der elektrischen Hirnaktivität bei den Testtieren, die ein statistisch signifikantes Niveau im Vergleich zur Applikation von Salzlösung (0.9 5 NaCl-Lösung) in allen Hirngebieten und bei delta, theta und alpha2 Frequenzen. Diese Muster korrelieren in signifikanter Weise mit den Mustern, die für Medikamente, wie sie bereits für die Behandlung von neurodegenerativen Erkrankungen (z.B. Tacrin: Ibach B und Haen E Acetylcholinesterase inhibition in Alzheimer's disease. Curr Pharm Des. (2004) 10(3):231-51); Werber EA and Rabey JM The beneficial effect of cholinesterase inhibitors on patients suffering from Parkinson's disease and dementia. Neural Transm (2001) 108: 1319-25, z.B. Piracetam: Flicker L und Grimlay Evans Piracetam for dementia or cognitive impairment. Cochrane Database Syst Rev (2001) 2:CD001011); Oepen G, Eisele K, Thoden U, Birg W Piracetam improves visuomotor and cognitive deficits in early Parkinsonism--a pilot study. Pharmacopsychiatry (1985) 18: 343-6, z.B. Memantine: Rabey JM, Nissipeanu P, Korczyn AD Efficacy of memantine, an NMDA receptor antagonist, in the treatment of Parkinson's disease. J Neural Transm Park Dis Dement Sect (1992) 4: 277-82), von Schmerz (z.B. Metamizol: Hackenthal E Paracetamol and metamizol in the treatment of chronic pain syndromes. Schmerz (1997) 11: 269-75) und nach Erleben eines Schlafanfalls (z.B. Amphetamin: Martinsson L und Eksborg S Drugs for stroke recovery: the example of amphetamines. Drugs Aging (2004) 21: 6 7-79) üblicherweise verwendet werden.; nicht jedoch mit Mustern, die bei Medikamenten für andere Indikationen auftreten.

Die beobachteten Frequenzänderungen wurden mittels einer Diskriminanzanalyse mit den Ergebnissen von Medikamenten zur Behandlung von Erkrankungen des Gehirns, insbesondere von neurodegenerativen Erkrankungen, Schmerz sowie nach Schlaganfall wie auch mit Medikamenten für andere Indikationen verglichen. Folgende Arzneimittel, welche bereits im selben Modell geprüft wurden, standen für Vergleichszwecke zur Verfügung:Memantine 1.0 mg; Tacrin 0.75 mg; Haloperidol 0.5 mg; Chlorpromazine 0.5 mg; Prothipendyl 1 mg; Clozapine 3 mg; Thioridazine 5 mg; Phentytoin 4 mg; Carbamazepine 15 mg; Valproinsäure 75 mg; Diazepam 0.5 mg; Methohexital 20 mg; Midazolam 0.5 mg; Meprobamat 60 mg; Phenobarbital 60 mg; Amphetamin 0.2 mg; Fenfluramin 1 mg; Kokain 10 mg; Mescaline 10 mg; Metanicotine 1mg; R-DOI 0.1 mg; R-DOM 0.2 mg; Dizocilpine 0.25 mg; LSD 0.05 mg; Acetylsalicylsäure 200 mg; Metamizol 100mg; Fentanyl 0.075 mg; Tramadol 10mg; Fluvoxamin 40 mg; Nomifensin 1mg; Mianserin 5 mg; Amitriptylin 10mg; Imipramin 10 mg; Die Angaben beziehen sich jeweils auf eine Dosierung in mg/kg Körpergewicht.

## Patentansprüche

1. Verwendung von Hesperidin (bzw. als Aglykon Hesperetin) oder seinen pharmazeutisch annehmbaren Salzen zur Herstellung eines Arzneimittels zur Behandlung von Erkrankungen des zentralen Nervensystems, insbesondere von neurodegenerativen Erkrankungen (.z.B. Morbus Alzheimer, Morbus Parkinson), Schmerz und der durch Schlaganfall verursachten Folgeschäden.

2. Verwendung von Hesperidin (bzw. als Aglykon Hesperetin) oder seinen pharmazeutisch annehmbaren Salzen zur Herstellung eines Nahrungsergänzungmittels zur Prophylaxe von Erkrankungen des zentralen Nervensystems, insbesondere von neurodegenerativen Erkrankungen (.z.B. Morbus Alzheimer, Morbus Parkinson).

3. Verwendung nach Anspruch 1oder 2, **dadurch gekennzeichnet, dass** das Nahrungsergänzungs- oder Arzneimittel zusätzlich pharmazeutisch annehmbare Träger, Hilfs- und/oder Verdünnungsmittel enthält.

4. Verwendung nach einem der Anspüche 1,2 oder 3, **dadurch gekennzeichnet, dass** das Nahrungsergänungs- oder Arzneimittel zur oralen Verabreichung hergerichtet wird.

## Geänderte Patentansprüche

### Geänderte Patentansprüche gemäss Regel 86(2) EPÜ.

1. Verwendung von Hesperidin (oder seinen pharmazeutisch annehmbaren Salzen) zur Herstellung eines Arzneimittels zur Prophylaxe und Behandlung von Morbus Alzheimer und Morbus Parkinson

2. Verwendung nach Anspruch 1, **dadurch gekennzeichnet, dass** das Arzneimittel zusätzlich pharmazeutisch annehmbare Träger, Hilfs- und/oder Verdünnungsmittel enthält

3. Verwendung nach einem der Anspüche 1 oder 2, **dadurch gekennzeichnet, dass** das Arzneimittel zur parenteralen, insbesondere auch subkutanen oder intramuskulären Verabreichung hergerichtet wird

4. Verwendung nach einem der Anspüche 1, 2 oder 3, **dadurch gekennzeichnet, dass** das Arzneimittel zur oralen Verabreichung hergerichtet wird, indem ein Enzymhemmer zugesetzt wird, der die Entstehung von Hesperetin durch Abspaltung des Zuckerrestes im Darm verhindert. Die Entstehung von Hesperetin kann auch auf andere Art verhindert werden (z.B. durch Einkapselung oder durch chemische Modifikation).
